(19) Europäisches Patentamt
European Patent Office
Office européen des brevets

(11) Veröffentlichungsnummer: **0 060 419**
**B1**

# (12) EUROPÄISCHE PATENTSCHRIFT

(45) Veröffentlichungstag der Patentschrift: **22.02.84**

(51) Int. Cl.$^3$: **C 07 C 49/825**, C 07 C 45/46

(21) Anmeldenummer: **82101480.0**

(22) Anmeldetag: **26.02.82**

(54) **Verfahren zur Herstellung von o- und p-Acylphenolen.**

(30) Priorität: **04.03.81 DE 3108076**

(43) Veröffentlichungstag der Anmeldung: **22.09.82 Patentblatt 82/38**

(45) Bekanntmachung des Hinweises auf die Patenterteilung: **22.02.84 Patentblatt 84/8**

(84) Benannte Vertragsstaaten: **BE CH DE FR GB LI**

(56) Entgegenhaltungen:
**US - A - 2 419 553**

**CHEMISCHES ZENTRALBLATT, Band I, Nr. 13, 28. März 1928, Berlin K.W. ROSENMUND et al. "Über Acylwanderungen an Phenolen" Seiten 1652 bis 1655**
**HOUBEN-WEYL "Methoden der Organischen Chemie" Band VII/2a, Teil 1 1973, GEORG THIEME VERLAG, Stuttgart**

(73) Patentinhaber: **BASF Aktiengesellschaft, Carl-Bosch-Strasse 38, D-6700 Ludwigshafen (DE)**

(72) Erfinder: **Mueller, Josef, Dr., Karl-Otto-Braun-Strasse 16, D-6700 Ludwigshafen (DE)**
Erfinder: **Wiersdorff, Walter-Wielant, Dr., Blockfeldstrasse 15, D-6704 Mutterstadt (DE)**
Erfinder: **Kirschenlohr, Werner, Dr., Alwin-Mittasch-Platz 10, D-6700 Ludwigshafen (DE)**
Erfinder: **Schwantje, Gerd, Dr., Theodor-Heuss-Strasse 2, D-6706 Wachenheim (DE)**

**EP 0 060 419 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von o- und p-Acylphenolen

Die Erfindung betrifft ein neues Verfahren zur Herstellung von o- und p-Acylphenolen durch Umsetzung von Phenol mit bestimmten Mengen von Säurehalogeniden und Aluminiumchlorid, mit Halogenbenzolen als Lösungsmittel bei 15 bis 55° C und dann oberhalb 55 bis 200° C.

Es ist bekannt, daß Chlorbenzol bereits bei Raumtemperatur mit $AlCl_3$ als Katalysator acyliert werden kann (Ber. 42 (1909), 1812; Bull. Soc. Chem. Belg. 61 (1952) 694–696).

Weiterhin ist bekannt, daß bei der Fries'schen Verschiebung ein Gemisch aus o- und p-Acylphenolen entsteht, wobei im allgemeinen der Anteil des p-Produktes stark überwiegt. Häufig verwendete Lösungsmittel sind $CS_2$, $CH_2Cl-CH_2-Cl$ und Nitrobenzol. Zwar kann die o-Selektivität durch Reaktion in Abwesenheit von Lösungsmitteln erhöht werden, jedoch ist diese Arbeitsweise technisch nur sehr schwierig zu realisieren, da bei hohen Umsätzen hochviskose, kaum noch rührbare Massen entstehen, deren Hydrolyse nicht ohne Schwierigkeiten durchgeführt werden kann (Ann. 460 (1928) 56–98).

Zudem sind unter den beschriebenen Reaktionsbedingungen die o-Acylphenole nur durch Wasserdampfdestillation zu isolieren, während die p-Produkte nur durch mehrmaliges Umkristallisieren rein zu erhalten sind.

Weiterhin sollen in Benzinen als Solventien hohe Selektivitäten an o-Verbindungen erreicht worden sein (Tetrahedron 20 (1964) 1661–1666).

Unter diesen Reaktionsbedingungen bilden sich jedoch ebenfalls zweiphasige, hochviskose nicht mehr rührbare Massen.

Es wurde nun gefunden, daß man o- und p-Acylphenole der Formel

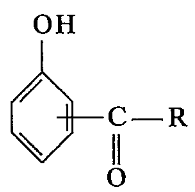 ,    I

worin der Acylrest in o- und/oder p-Stellung zur Hydroxygruppe steht und R einen aliphatischen, cycloaliphatischen, araliphatischen Rest bedeutet, durch Umsetzung von aromatischen Verbindungen mit Säurehalogeniden in Anwesenheit von Aluminiumchlorid, vorteilhaft erhält, wenn man Phenol mit einem Säurehalogenid der Formel

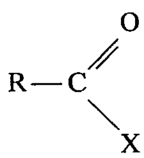    II

worin R die vorgenannte Bedeutung besitzt und X ein Halogenatom bezeichnet, in einer Menge von 0,5 bis 1,5 je Mol Phenol in Gegenwart von 0,5 bis 1,5 Mol Aluminiumchlorid je Mol Phenol und von Halogenbenzolen als Lösungsmittel zuerst bei einer Temperatur von 15 bis 55° C und dann von oberhalb 55 bis 200° C umsetzt.

Die Umsetzung kann für den Fall der Verwendung von Propionylchlorid durch die folgenden Formeln wiedergegeben werden:

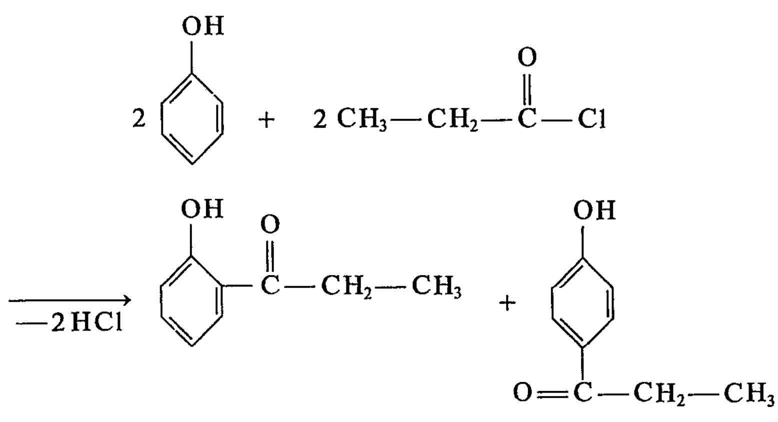


Im Hinblick auf die bekannten Verfahren liefert das Verfahren nach der Erfindung auf einfacherem und wirtschaftlicherem Wege o- und p-Acylphenole in besserer Ausbeute, Reinheit und Raum-Zeit-

Ausbeute. Die p-Endstoffe I kristallisieren in höheren Reinheiten nach Zugabe von Eiswasser aus; die in den Mutterlaugen befindlichen o-Derivate erhält man durch einfache Destillation in guter Reinheit. Schwierigkeiten in der Manipulation (Rühren) des Reaktionsgemisches treten nicht auf. Alle diese vorteilhaften Ergebnisse sind im Hinblick auf den Stand der Technik überraschend. Es konnte mit den erfindungsgemäßen Maßnahmen nicht erwartet werden, daß die Acylierung von Halogenbenzolen, die Bildung entsprechender halogenierter Nebenprodukte oder erhöhte Estermengen nicht in deutlichem Maße auftreten.

Man setzt den Ausgangsstoff II mit Phenol in stöchiometrischer Menge oder im Überschuß jeder Komponente zur anderen, im Verhältnis von 0,5 bis 1,5 Mol, vorzugsweise 0,95 bis 1,1 Mol Ausgangsstoff II je Mol Phenol um. Bevorzugte Ausgangsstoffe II und dementsprechend bevorzugte Endstoffe I sind solche, in deren Formeln der Acylrest in o- und/oder p-Stellung zur Hydroxygruppe steht und $R^1$ einen Alkylrest mit 1 bis 6 Kohlenstoffatomen, einen Cycloalkylrest mit 5 bis 8 Kohlenstoffatomen, einen Aralkylrest mit 7 bis 12 Kohlenstoffatomen bedeutet und X ein Bromatom oder insbesondere ein Chloratom bezeichnet. Die vorgenannten Reste können noch durch unter den Reaktionsbedingungen inerte Gruppen, z. B. Alkyl- oder Alkoxygruppen mit jeweils 1 bis 4 Kohlenstoffatomen substituiert sein.

Es kommen beispielsweise folgende Ausgangsstoffe II in Betracht: Acetylchlorid, Propionsäurechlorid, Buttersäurechlorid, Isobuttersäurechlorid, Valeriansäurechlorid, Isovaleriansäurechlorid, Capronsäurechlorid, Önanthsäurechlorid, Caprylsäurechlorid; entsprechende Bromide.

Die Umsetzung wird in Gegenwart von 0,5 bis 2, vorteilhaft 0,95 bis 1,1 Mol Aluminiumchlorid je Mol Phenol umgesetzt. Als Lösungsmittel kommen bevorzugt Monobrom-, Monochlor-, Dibrom- und Dichlorbenzole in Betracht. Zweckmäßig verwendet man 100 bis 10 000, vorteilhaft 130 bis 200 Gewichtsprozent Lösungsmittel, bezogen auf die Gewichtsmenge Phenol. Die Umsetzung wird in 2 Temperaturstufen, zuerst bei 15 bis 55° C, vorteilhaft 40 bis 51° C und dann bei oberhalb 55 bis 200° C, zweckmäßig bei 56 bis 200° C, vorteilhaft 80 bis 130° C, drucklos oder unter Druck, kontinuierlich oder diskontinuierlich durchgeführt.

Die Reaktion kann in folgender Weise erfolgen: Ein Gemisch von Phenol, Säurechlorid, Halogenbenzol, Aluminiumchlorid wird während 0,2 bis 1 Stunden bei der Reaktionstemperatur der 1. Stufe, dann während 0,5 bis 3 Stunden bei der Reaktionstemperatur der Stufe 2 gehalten. Dann wird der Endstoff I in üblicher Weise, z. B. durch Zugabe von Eiswasser, Filtration des p-Endstoffs I, fraktionierte Destillation und Abtrennung der o-Komponente I, isoliert.

Die nach dem erfindungsgemäßen Verfahren herstellbaren Verbindungen sind wertvolle Ausgangsstoffe für die Herstellung von Pharmazeutika, Schädlingsbekämpfungsmitteln und Farbstoffen. Bezüglich der Verwendung wird auf vorgenannte Literatur verwiesen.

Die in den folgenden Beispielen genannten Teile sind Gewichtsteile.

## Beispiel 1

### o- und p-Hydroxypropionphenon

Zu einer Mischung von 940 Teilen Phenol in 1500 Teilen Chlorbenzol werden 1350 Teile $AlCl_3$ so zudosiert, daß die Temperatur 40° C nicht überschreitet. Dann gibt man langsam während 60 Minuten 1017 Teile Propionylchlorid so zu, daß 50° C nicht überschritten werden. Darauf erhitzt man auf 105° C und rührt 2 Stunden bei dieser Temperatur nach. Nach Zugabe von 10 000 Teilen Eiswasser trennt man den ausgefallenen p-Endstoff I durch Filtration ab. Man trennt die Phasen, zieht das Chlorbenzol im Vakuum ab und destilliert den o-Endstoff I bei 30 mbar und 125 bis 133° C.

Ausbeute:

o-Hydroxypropionphenon: 1083 Teile (68% der Theorie), $Kp_{20\ mbar}$ 115–117° C
p-Hydroxypropionphenon: 330 Teile (22% der Theorie) vom Fp 148° C.

## Beispiel 2

Analog Beispiel 1 werden hergestellt:

830 Teile o-Hydroxyacetophenon ($Kp_{30\ mbar}$ 115–119° C) (61% der Theorie)
381 Teile p-Hydroxyacetophenon (F: 104–105° C) (28% der Theorie).

## Beispiel 3

Zu einer Mischung von 188 Teilen Phenol in 600 Teilen o-Dichlorbenzol werden 534 Teile $AlCl_3$ so zudosiert, daß die Temperatur 40° C nicht überschreitet. Dann gibt man langsam während 60 Minuten

185 Teile Propionylchlorid so zu, daß 50° C nicht überschritten werden. Darauf heizt man innerhalb von einer Stunde auf 105° C auf und rührt noch 15 Minuten nach. Nach Zugabe von 2500 Teilen Eiswasser trennt man den ausgefallenen p-Endstoff I durch Filtration ab. Der Feststoff wird mit Toluol nachgewaschen und getrocknet. Die vereinigten organischen Phasen werden destilliert.

Ausbeute:
p-Hydroxypropionphenon 236 Teile (79% der Theorie) vom Fp 148° C
o-Hydroxypropionphenon 55 Teile (18% der Theorie) $Kp_{20\,mbar}$ 115—118° C.

Beispiel 4

(Vergleichsbeispiel)

Zu 136 Teilen Phenylacetat werden 133,5 Teile $AlCl_3$ portionsweise zugefügt und anschließend eine Stunde auf 170° C erhitzt. Dabei bildete sich eine hochviskose, nicht mehr rührbare dunkle Masse, die nach Abkühlen mit 1000 Teilen Eiswasser zersetzt wird. Die Mischung wird mit Wasserdampf destilliert. Anschließende Reindestillation lieferte 36 Teile (26,4% der Theorie) o-Hydroxyacetophenon. Das p-Produkt ließ sich nicht in reiner Form isolieren. Mehrmaliges Nacharbeiten ergab Ausbeuten zwischen 22 und 30% an o-Hydroxyacetophenon.

Beispiel 5

(Vergleichsbeispiel)

133,5 Teile Aluminiumchlorid werden in 300 Teilen n-Heptan suspendiert und 94 Teile Phenol zugefügt. Bei 22° C werden unter Rühren 78,5 Teile Acetylchlorid langsam zugegeben. Darauf wird auf Rückfluß erhitzt. Sobald ein Umsatz von 30% der Theorie erreicht war, muße die Reaktion abgebrochen werden, da wegen der hohen Viskosität ein Rühren nicht mehr möglich war.

Beispiel 6

(Vergleichsbeispiel)

Eine Mischung aus 133,5 Teilen $AlCl_3$ und 300 Teilen Chlorbenzol wird auf 100° C aufgeheizt und 136 Teile Phenylacetat so zugegeben, daß die Temperatur nicht über 110° C ansteigt (0,5 Stunden). Nach beendeter Zugabe wird noch eine Stunde bei dieser Temperatur nachgerührt und zur Hydrolyse 1000 Teile Eiswasser gegeben. Es werden 33 Teile (24% der Theorie) p-Hydroxyacetophenon isoliert. Nach Destillation der Mutterlauge erhält man insgesamt 87 Teile Reaktionsgemisch folgender Gesamtausbeute (GC-Bestimmung):

Phenol (18,4% der Theorie),
o-Hydroxyacetophenon (34% der Theorie),
p-Hydroxyacetophenon (24% der Theorie),
p-Chloracetophenon (22,6% der Theorie),
sonstige Nebenprodukte (1% der Theorie).

**Patentanspruch**

Verfahren zur Herstellung von o- und p-Acylphenolen oder Formel

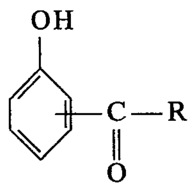

I

worin der Acylrest in o- und/oder p-Stellung zur Hydroxygruppe steht und R einen aliphatischen, cycloaliphatischen, araliphatischen Rest bedeutet, durch Umsetzung von aromatischen Verbindungen mit Säurehalogeniden in Anwesenheit von Aluminiumchlorid, dadurch gekennzeichnet, daß man Phenol mit einem Säurehalogenid der Formel

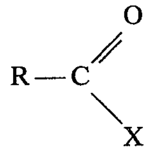

II

worin R die vorgenannte Bedeutung besitzt und X ein Halogenatom bezeichnet, in einer Menge von 0,5 bis 1,5 Mol je Mol Phenol in Gegenwart von 0,5 bis 1,5 Mol Aluminiumchlorid je Mol Phenol und von Halogenbenzolen als Lösungsmittel zuerst bei einer Temperatur von 15 bis 55° C und dann von oberhalb 55 bis 200° C umsetzt.

**Claim**

A process for the preparation of o-acylphenols and p-acylphenols of the formula

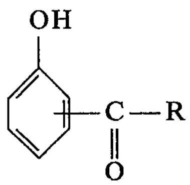

I

where the acyl radical is in the o-position and/or p-position to the hydroxyl group and R is an aliphatic, cycloaliphatic or araliphatic radical, by reacting aromatic compounds with acid halides in the presence of aluminium chloride, wherein phenol is reacted with an acid halide of the formula

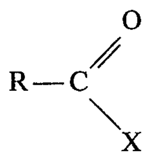

II

where R has the above meaning and X is halogen, in an amount of from 0.5 to 1.5 moles per mole of phenol, in the presence of from 0.5 to 1.5 moles of aluminium chloride per mole of phenol, and of a halobenzene as the solvent, initially at from 15 to 55° C and then at from 55 to 200° C.

**Revendication**

Procédé de préparation de o- et de p-acyl-phénols de la formule

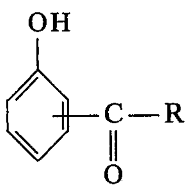

I

dans laquelle le groupe acyle se trouve en position ortho et (ou) para par rapport au groupe oxhydryle et R désigne un reste aliphatique, cycloaliphatique ou araliphatique, par réaction de composés aromatiques en présence de chlorure d'aluminium avec des halogénures d'acides, caractérisé en ce que l'on fait réagir du phénol avec un halogénure d'acide de la formule

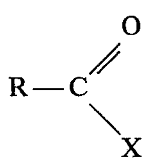

II

dans laquelle R possède la signification définie et X désigne un atome d'halogène, en une proportion de 0,5 à 1,5 mole par mole de phénol, en présence de 0,5 à 1,5 mole de chlorure d'aluminium par mole de phénol, dans un halobenzène servant de solvant, d'abord à une température comprise entre 15 et 55° C, puis à une température supérieure à 55° C et pouvant aller jusqu'à 200° C.